(19) **Europäisches Patentamt / European Patent Office / Office européen des brevets**

(11) **EP 4 218 146 B1**

(12) **FASCICULE DE BREVET EUROPEEN**

(45) Date de publication et mention de la délivrance du brevet:
**05.08.2026 Bulletin 2026/32**

(21) Numéro de dépôt: **21798064.8**

(22) Date de dépôt: **24.09.2021**

(51) Classification Internationale des Brevets (IPC):
*H04B 5/79* ^(2024.01)    *H04B 5/26* ^(2024.01)
*H02J 50/12* ^(2016.01)    *A61N 1/378* ^(2006.01)
*A61N 1/372* ^(2006.01)    *H02J 7/64* ^(2026.01)

(52) Classification Coopérative des Brevets (CPC):
**H04B 5/79; A61N 1/37223; A61N 1/3787; H02J 50/12; H04B 5/266;** H02J 7/64; H02J 2105/46

(86) Numéro de dépôt international:
**PCT/FR2021/051643**

(87) Numéro de publication internationale:
**WO 2022/064153 (31.03.2022 Gazette 2022/13)**

(54) **DISPOSITIF MÉDICAL IMPLANTABLE ACTIF**

**AKTIVE IMPLANTIERBARE MEDIZINISCHE VORRICHTUNG**

**ACTIVE IMPLANTABLE MEDICAL DEVICE**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **25.09.2020 FR 2009787**

(43) Date de publication de la demande:
**02.08.2023 Bulletin 2023/31**

(73) Titulaires:
• **Neurinnov**
  **34000 Montpellier (FR)**
• **Centre national de la recherche scientifique**
  **75016 Paris (FR)**
• **INRIA - Institut National de Recherche en Informatique et en Automatique**
  **78150 Le Chesnay (FR)**
• **Université de Montpellier**
  **34090 Montpellier (FR)**

(72) Inventeurs:
• **ANDREU, David**
  **34570 Montarnaud (FR)**
• **GUIRAUD, David**
  **34000 Montpellier (FR)**
• **SORLI, Brice**
  **34530 Montagnac (FR)**
• **VENA, Arnaud**
  **34270 Saint-Mathieu-de-Tréviers (FR)**

(74) Mandataire: **Icosa**
  **83 avenue Denfert-Rochereau**
  **75014 Paris (FR)**

(56) Documents cités:
FR-A1- 3 085 551    US-A1- 2015 209 591
US-A1- 2018 034 319

**Description**

## DOMAINE DE L'INVENTION

La présente invention concerne les dispositifs médicaux, plus précisément les dispositifs médicaux implantables actifs (DMIA).

## ÉTAT DE LA TECHNIQUE

De manière classique et bien connue en soi, un dispositif médical implantable actif (DMIA) est défini comme un dispositif dont le fonctionnement dépend d'une source d'énergie autre que celle générée par le corps humain, et qui agit sur cette énergie fournie en en modifiant la densité ou en la convertissant. Un DMIA peut être destiné à être partiellement ou complètement introduit dans le corps humain, de manière à restaurer une fonction vitale, compenser une déficience et/ou mesurer des paramètres physiologiques. Classiquement, un DMIA comprend ainsi au moins un élément actif destiné à être partiellement introduit dans le corps humain par intervention clinique et destiné à rester en place après la procédure pendant au moins 30 jours. Au sens large, il est considéré qu'un DMIA permet de transférer de l'énergie et de l'information de part et d'autre de la peau d'un patient implanté. Il est connu de l'état de la technique que la meilleure option, actuellement, pour gérer le transfert d'énergie et/ou d'informations des implants, est l'option WPT (*wireless power transfer*) se basant sur des transferts de champs électromagnétiques. Ainsi, la plupart des dispositifs implantés comportent deux bobines, la première située à l'extérieur du corps d'un sujet implanté et la deuxième située à l'intérieur du corps du sujet implanté. Ce transfert est donc effectué par liaison sans-fil (inductive), à travers la peau et en champ proche, plus particulièrement via la technologie NFC (HF à 13.56 MHz, selon les standards ISO 14443 ou ISO 15693). La technologie NFC, permet de réduire la taille des bobines. L'acronyme « NFC » signifie *« Near Field Communication »* et a son acronyme équivalent en français : « CCP » pour « communication en champ proche ». Il s'agit d'une technologie de communication sans fil à courte portée et à haute fréquence. Cette technologie, bien connue en soi, permet l'échange d'informations entre des périphériques, généralement une puce NFC et un lecteur NFC, jusqu'à une distance d'environ 10 cm. Cette technologie est une extension de la norme ISO/CEI 14443 standardisant les cartes de proximité utilisant la radio-identification (RFID) qui combinent une carte à puce et un lecteur au sein d'un seul périphérique. De manière connue de l'état de la technique, les puces NFC sont notamment composées d'une antenne et d'un circuit intégré et présentent un étage d'entrée contenant un circuit de protection (comportant notamment au moins une diode Zener) destiné à protéger la puce NFC, et menant, de fait, à limiter la tension développée dans l'antenne. De manière connue de l'état de la technique, cette caractéristique intrinsèque aux puces NFC limite l'énergie récupérable par d'autres éléments fonctionnels de l'implant. En effet, la limitation intrinsèque à la technologie est que la puce NFC, passive dans le cas présent, restitue peu de l'énergie qu'elle reçoit, induisant un rendement très faible (environ 15 mW récupéré pour 500 mW émis).

Ces observations peuvent s'étendre à toute forme de puce comportant une antenne destinée à recevoir de l'énergie et/ou de l'information.

La demande de brevet états-unienne US2018034319 décrit un dispositif de transfert/collecte d'énergie comprenant un premier module et un second module. Le premier module est destiné à transférer de l'énergie et des données au second module, via une antenne.

La demande de brevet française FR3085551 décrit des transpondeurs passifs sans contact, pour lesquels se pose un problème de protection lorsqu'ils ont mis en présence de chargeurs sans contact.

De plus, dans le contexte particulier d'un dispositif implantable, le transfert d'énergie et/ou d'informations est fortement atténué par les tissus de la personne équipée du dispositif. Cette atténuation conduit à plusieurs contraintes. D'une part, les signaux émis doivent avoir une intensité suffisante pour être reçus correctement par le récepteur. D'autre part, le transfert d'énergie doit être limité pour ne pas endommager les tissus - conformément aux limites réglementaires de débit d'absorption spécifique (DAS). Ce compromis est mal pris en compte dans l'état de la technique.

L'objectif de l'invention est de transmettre via un lien inductif énergie et données à un dispositif implanté dans le corps, à partir d'un dispositif externe en optimisant l'énergie récupérée tout en maitrisant la portée de l'implant.

La présente invention atteint l'objectif susmentionné en proposant l'ajout d'un montage électronique comportant une antenne associée à un circuit interne, pouvant être une puce NFC, pour permettre d'atteindre une tension développée par l'antenne réceptrice au-delà de limites imposées par le circuit interne.

## RÉSUMÉ

L'invention concerne donc un dispositif médical implantable actif défini par la revendication 1.

L'invention permet de transmettre énergie et données à un dispositif implanté dans le corps via un lien transcutané basé sur une technologie comprenant une antenne associée à un circuit intégré, comme par exemple la technologie NFC, l'invention permettant d'adapter l'impédance du circuit intégré (et de l'antenne associée) de manière à optimiser l'énergie récupérée. Cette adaptation d'impédance repose ainsi sur l'ajout d'un montage électronique au circuit intégré (par exemple celui d'une puce NFC) pour permettre à l'antenne associée d'atteindre une tension au-delà des limites imposées par le circuit intégré et d'optimiser le rendement du transfert d'énergie entre le module externe et le module interne du dispositif.

Le dispositif selon l'invention peut comprendre une ou plusieurs des caractéristiques suivantes, prises isolément les unes des autres ou en combinaison les unes avec les autres :

- le rapport $\dfrac{C_3}{C_3+C_4}$ est supérieur ou égal à 0.25, de préférence compris entre 0.3 et 0.6
- le module externe comporte une unique antenne externe, et en ce que le module interne comporte une unique antenne interne,
- chaque antenne est reliée à un circuit d'adaptation d'impédance et que chaque antenne et son circuit d'adaptation d'impédance associé résonnent à une fréquence comprise entre 12 et 14 MHz, plus précisément 13,56MHz,
- le module externe et le module interne sont destinés à transférer de l'énergie et des données de l'un vers l'autre de manière simultanée,
- le module externe et le module interne sont destinés à transférer des données de l'un vers l'autre de manière bidirectionnelle,
- le module externe et le module interne sont destinés à transférer de l'énergie et des données de l'un vers l'autre sur une unique bande de fréquences.

L'invention a également pour objet un procédé de transfert d'énergie et de données, le procédé étant mis en œuvre au moyen d'un dispositif selon l'une des caractéristiques énoncées ci-dessus, la tension fournie au circuit intégré interne étant comprise entre 100mV et 5V, la tension fournie au montage électronique de récupération d'énergie étant comprises entre 100mV et 50V.

**DESCRIPTION DÉTAILLÉE**

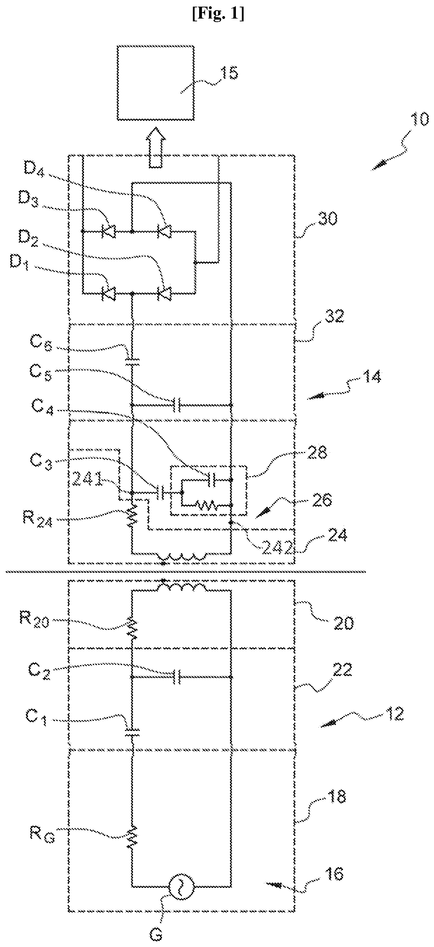

La présente invention concerne donc un dispositif médical implantable actif (DMIA) 10, plus particulièrement un lien inductif pour DMIA 10, apte à être implanté dans un sujet, tel que schématiquement illustré en figure 1. Plus particulièrement, le dispositif 10 comprend deux modules de communication et de transfert d'énergie 12, 14 complémentaires l'un de l'autre (un module externe 12 et un module interne 14) et un stimulateur 15. En lieu et place d'un stimulateur, certains modes de réalisation peuvent, par exemple, présenter un dispositif de mesure de signaux physiologiques ou physiques internes au corps humain, parmi lesquels l'EMG (électromyogramme), l'EEG (électroencéphalogramme), l'ECG (électrocardiogramme), ou l'ENG (électroneurogramme) ou mesure de la température.

Lorsque le dispositif 10 est mis en œuvre pour le sujet, le module externe 12 est positionné à l'extérieur du sujet, par exemple fixe sur la peau du sujet, et le module interne 14 est implanté à l'intérieur du sujet, par exemple sous la peau du sujet. Le stimulateur 15 est connecté au module interne 14 et se retrouve donc, après implantation, également positionné à l'intérieur du sujet. Dans le mode de réalisation comportant un stimulateur 15, ce stimulateur 15 peut être destiné à stimuler des tissus, nerfs ou muscles par exemple, afin de contrôler des organes ou des mouvements de membres du corps.

Le module externe 12 et le module interne 14 sont destinés à transférer de l'énergie et des données de l'un vers l'autre. L'énergie est ensuite transférée vers le stimulateur 15. Plus particulièrement, le transfert d'énergie se fait de manière unidirectionnelle du module externe 12 vers le module interne 14, et le transfert de données peut être bidirectionnel, du module externe 12 vers le module interne 14 et vice versa.

Selon le mode de réalisation illustré en figure 1, le module externe 12 est un lecteur NFC 16 comprenant :

- un générateur 18 modélisé, sur la figure 1, par un générateur sinusoïdal G de fréquence 13,56MHz et la résistance $R_G$ associée,
- une antenne externe 20 (et la résistance $R_{20}$ associée) connectée au générateur 18,
- un réseau externe d'adaptation d'impédance 22 situé entre le générateur 18 du lecteur NFC 16 et l'antenne externe 20, le réseau externe d'adaptation d'impédance 22 comportant un premier condensateur de capacité $C_1$ et un deuxième condensateur de capacité $C_2$ branchés de manière à former un pont diviseur capacitif.

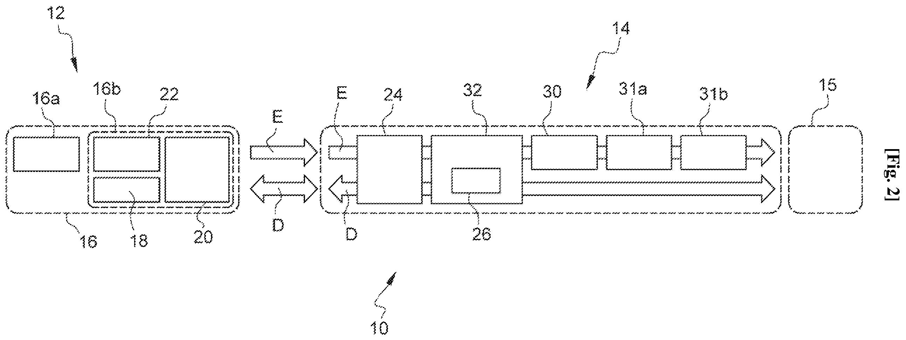

Comme illustré sur la figure 2, le générateur 18, l'antenne externe 20 et le réseau externe d'adaptation d'impédance 22 forment la partie analogique 16b du lecteur NFC 16. En outre, le lecteur NFC16 peut comprendre une partie digitale 16a liée à la partie analogique 16b par un convertisseur digital/analogique.

L'utilisation d'un lecteur NFC permet un échange de données dans deux directions. Les données échangées concernent :

- des ordres et paramètres de stimulation du module externe 12 vers le module interne 14,
- des réponses, notifications d'erreurs et données mesurées retournées du module interne 14 vers le module externe 12.

De manière bien connue en soi, l'adaptation d'impédance est une technique permettant d'optimiser le transfert d'une puissance électrique entre un émetteur et un récepteur électrique pour optimiser l'échange de données et/ou d'énergie entre le récepteur et l'émetteur, notamment l'échange de signaux de télécommunications.

Le module interne 14 comprend :

- une antenne interne 24 (et la résistance $R_{24}$ associée) définissant deux bornes (241, 242),
- une puce 26 destinée à coopérer avec le lecteur NFC 16 du module externe 12, la puce 26 étant connectée à l'antenne interne 24, la puce 26 comportant par ailleurs l'association en série entre les bornes 241 et 242 de l'antenne interne 24 :

> o d'un troisième condensateur de capacité $C_3$ et
> o d'un circuit intégré interne 28 présentant une impédance d'entrée équivalente Z dont la partie capacitive est modélisée par un quatrième condensateur de capacité $C_4$, et, le rapport $\frac{C_3}{C_3+C_4}$ étant supérieur ou égal à 0.1, $C_3$ et $C_4$ formant un pont diviseur capacitif,

- un montage électronique de récupération d'énergie électrique (non référencé) connecté à l'antenne interne 24 et destiné à pourvoir le simulateur 15 en énergie électrique, le montage électronique de récupération étant externe au circuit intégré interne 28 de la puce 26,
- le montage électronique de récupération d'énergie comportant un circuit de redressement 30 situé en amont d'un montage électronique de stockage d'énergie et un circuit de régulation de tensions (non représentés), le circuit de redressement 30 comportant plusieurs diodes $D_1$, $D_2$, $D_3$, $D_4$ destinées à générer une source de tension continue destinée à alimenter le montage électronique de récupération d'énergie,
- un réseau interne d'adaptation d'impédance 32 situé entre l'antenne interne 24 et le circuit de redressement 30, ce réseau interne d'adaptation d'impédance 32 comportant un cinquième et un sixième condensateurs respectivement de capacités $C_5$, $C_6$ branchés de manière à former un réseau en L, permettant d'accorder l'impédance.

Le circuit intégré interne 28 est de préférence un circuit de type NFC.

En outre, comme illustré sur la figure 2, un module de micro-stockage 31a et un module de régulation de tension 31b peuvent être disposés entre le circuit de redressement 30 et le stimulateur 15.

Dans le mode de réalisation illustré en figure 1, la valeur de la résistance RG est de 50Ω, les résistances R20 et R24 sont de 5Ω. Par ailleurs, la valeur de la première capacité C1 est de 38pF, celle de la deuxième capacité C2 est de 62pF, et celle de la capacité C3 est de 36pF. La valeur de la quatrième capacité C4 est de 35pF, celle de la cinquième capacité C5 est de 6,8pF et la valeur de la sixième capacité C6 est de 8,2pF. Dans d'autres modes de réalisation, les valeurs des différentes capacités peuvent légèrement varier, restant toutefois du même ordre de grandeur. Dans ce mode de réalisation, le rapport $\frac{C_3}{C_3+C_4}$ est d'environ 0.5, ce qui conduit à une bonne répartition du signal reçu par l'antenne interne 24 entre la puce 26 d'une part et le montage électronique de récupération d'énergie d'autre part.

Dans le cas de la présente invention, le module externe 12 comporte une unique antenne externe 20, et le module interne 14 comporte une unique antenne interne 24. Chaque antenne 20, 24 est à la fois une antenne réceptrice et une antenne émettrice. Plus particulièrement, l'antenne externe 20 est une antenne émettrice d'énergie et de données, et, potentiellement, une antenne réceptrice de données, et l'antenne interne 24 est une antenne réceptrice de données et d'énergie et, potentiellement, une antenne émettrice de données. Les antennes externe et interne 20, 24 sont conçues pour obtenir une valeur d'inductance qui permet de résonner à une fréquence comprise entre 12 et 14 MHz, plus précisément à une fréquence de 13,56 MHz (*fréquence dite ISM*), compte tenu de l'impédance d'entrée équivalente Z du circuit intégré interne 28 de la puce 26.

Grâce à ces deux antennes 20, 24, le module externe 12 et le module interne 14 transfèrent de l'énergie et des données de l'un vers l'autre de manière simultanée, et sur une unique bande de fréquences. De plus, comme déjà mentionné, le module externe 12 et le module interne 14 peuvent transférer des données de l'un vers l'autre de manière bidirectionnelle.

Comme déjà indiqué en introduction, le circuit intégré interne 28 de la puce 26 présente un étage d'entrée contenant un circuit de protection (comportant notamment au moins une diode Zener) menant à limiter la tension développée aux bornes de l'antenne interne 24. Dans un mode de réalisation alternatif, la protection peut être obtenue au moyen d'une cascade de diodes classiques. Ceci limite l'énergie récupérable par le montage de récupération d'énergie électrique destiné à alimenter le stimulateur 15. L'ajout du condensateur $C_3$ connecté en série permet de limiter la tension fournie au circuit intégré interne 28 de la puce 26 tout en maintenant une tension élevée à la borne d'entrée du montage électronique de récupération d'énergie électrique, permettant ainsi un bon approvisionnement énergétique du stimulateur 15.

Le rapport $\frac{C_3}{C_3+C_4}$ exprime la répartition de l'énergie entre le montage électronique de récupération d'énergie électrique et le circuit intégré interne 28 de la puce 26. En l'absence de capacité $C_3$, le rapport est zéro et les transferts d'énergie sont très limités. Il a été observé que pour un rapport $\frac{C_3}{C_3+C_4}$ supérieur à 0.1, le transfert d'énergie est nettement amélioré. De préférence, le rapport $\frac{C_3}{C_3+C_4}$ est supérieur à 0.25, ou même supérieur à 0.3. Le rapport $\frac{C_3}{C_3+C_4}$ est inférieur à 0.7. Si le rapport tend vers 0 ($C_3$ est petit devant $C_4$), tout se passe comme s'il n'y avait plus de pont diviseur capacitif et la diode Zener du circuit intégré interne 28 de la puce 26 se remet à limiter la tension d'entrée du circuit de récupération d'énergie électrique. Dans le cas contraire, pour un rapport tendant vers 1 ($C_3$ est grand devant $C_4$), le circuit intégré interne 28 de la puce 26 ne reçoit plus de signal et tout est transféré au circuit de récupération d'énergie, selon :

$$V_{nfc} = \frac{C_3}{(C_3 + C_4)} * V_{antenne}$$

La recherche d'un transfert maximal d'énergie est typiquement recherchée dans les chargeurs inductifs, mais n'est pas souhaitable dans le contexte d'un dispositif implantable pour lequel l'énergie transférable est limitée du fait de la sensibilité des tissus et pour lequel l'intensité du signal - transmis en même temps que l'énergie - doit être suffisante pour être reçu correctement.

Dans un mode de réalisation, le rapport $\frac{C_3}{C_3+C_4}$ est compris entre 0.3 et 0.6.

Dans cette configuration, du point de vue du montage électronique de récupération d'énergie électrique, le circuit intégré interne 28 de la puce 26 est considérée comme un condensateur en parallèle avec une résistance variable dont la valeur dépend de la tension d'entrée, de manière à modéliser l'effet Zener d'un circuit NFC réel par exemple, faisant partie du réseau d'adaptation d'impédance entre l'antenne interne 24 et le montage électronique de récupération d'énergie électrique.

Ainsi, dans la présente invention, l'ensemble formé par le troisième condensateur $C_3$, le circuit intégré interne 28 de la puce 26 et le réseau interne d'adaptation d'impédance 32 assure à la fois :

- l'accord d'impédance pour optimiser le couplage entre l'antenne interne 24 et le montage électronique de récupération d'énergie électrique,
- la déviation d'une partie de l'énergie reçue, de manière à assurer une alimentation énergétique stable et suffisante pour le montage électronique de récupération d'énergie électrique sans affecter le fonctionnement de la puce 26.

La particularité du lien transcutané de la présente invention est ainsi de transférer simultanément de l'énergie et des données (les données étant en plus transférées de manière bidirectionnelle) par un seul et même lien en champ proche, et donc une seule et même antenne de chaque côté de la peau du sujet, en récupérant une quantité d'énergie optimisée au niveau du module interne 14.

Les avantages de la présente invention par rapport à l'état de l'art sont :

- le transfert d'énergie s'effectue en même temps que la communication, sans contraintes mutuelles,
- le dispositif 10 ne comporte qu'un seul jeu d'antennes 20, 24, à savoir une seule antenne de chaque côté de la peau du sujet, évitant l'utilisation de plusieurs antennes et réduisant ainsi grandement l'encombrement stérique,
- l'énergie et les données sont transmises sur une seule et même bande de fréquence,
- le dispositif 10 est compatible avec un système de lecture NFC standard,
- la portée du transfert de données est réduite, contribuant à limiter les risques d'écoute et d'intrusion et garantissant ainsi une meilleure sûreté de transmission.

En particulier, le fait de n'utiliser qu'un seul jeu d'antennes 20, 24 permet de concilier échange d'informations et d'énergie ce qui rompt avec l'état de la technique : en effet, de manière classique les dispositifs comportent deux systèmes séparés (l'un pour les informations, l'autre pour l'énergie) alors que l'approche de la présente invention permet de fusionner les deux, réduisant de fait l'encombrement. Ce résultat est obtenu sans complexification du dispositif vu qu'il ne s'agit que de l'ajout de quelques composants passifs.

Plusieurs implants de l'état de la technique ne comportent qu'une seule antenne, toutefois, lesdites antennes ne fonctionnent pas dans cette bande de fréquence et pas NFC (HF à 13.56 MHz, selon les standards ISO 14443 ou ISO 15693) avec récupération d'énergie. Le dispositif 10 selon l'invention permet de mettre en œuvre un procédé de transfert transcutané d'énergie et de données dans lequel la tension fournie au circuit intégré interne 28 est comprise entre 100mV et 5V, et la tension fournie au montage électronique de récupération d'énergie est comprise entre 100mV et 50V. Le rendement de transfert énergétique entre le module externe 12 et le module interne 14 est ainsi compris entre 10% et 70%. Le débit de transfert entre le module externe 12 et le module interne 14 est par ailleurs compris entre 6kbits/s et 900kbits/s, préférentiellement entre 106kbit/s et 847kbit/s ou entre 6,62kbits/s et 26,48kbits/s, de manière à Si l'on souhaite également couvrir la norme *ISO/IEC 15693*.

Le point essentiel de la présente invention réside donc dans l'augmentation de l'efficacité du transfert d'énergie transcutané en associant un circuit intégré, par exemple une puce NFC standard, avec un montage électronique de récupération d'énergie externe au circuit intégré, de manière à obtenir un dispositif 10 qui combine un bon rendement de transfert énergétique avec un lien de communication sans fil fiable.

**DESCRIPTION DES FIGURES**

**[Fig. 1] Figure 1** est un schéma électronique d'un dispositif selon la présente invention,
**[Fig. 2] Figure 2** est un schéma fonctionnel d'un dispositif selon la présente invention.

## Revendications

1. Dispositif médical implantable actif (10) apte à être partiellement implanté dans un sujet, le dispositif comprenant, une fois implanté, un module externe (12) au sujet et un module interne (14) au sujet, le module externe (12) et le module interne (14) étant destinés à transférer des données de l'un vers l'autre et de l'énergie du module externe (12) vers le module interne (14),

   - le module externe (12) comportant une antenne externe (20),
   - le module interne (14) comportant :

     ▪ une antenne interne (24) définissant deux bornes (241, 242);
     ▪ un circuit intégré interne (28) associé à l'antenne interne (24) ayant une impédance d'entrée équivalente de partie capacitive $C_4$, ledit circuit intégré interne (28) étant connecté à la première borne (242) et étant connecté à un condensateur de capacité $C_3$, ledit condensateur de capacité $C_3$ étant connecté à la seconde borne (241) formant ainsi une association en série du circuit intégré interne (28) et du condensateur $C_3$ entre les deux bornes (241, 242), avec le rapport $\frac{C_3}{C_3+C_4}$ supérieur ou égal à 0.1 et inférieur à 0.7, et
     ▪ un montage électronique de récupération d'énergie électrique connecté auxdites bornes (241, 242), le montage électronique de récupération étant externe au circuit intégré interne (28).

2. Dispositif (10) selon la revendication précédente, **caractérisé en ce que** le rapport $\frac{C_3}{C_3+C_4}$ est supérieur ou égal à 0.25, de préférence compris entre 0.3 et 0.6.

3. Dispositif (10) selon l'une quelconque des revendications précédentes, **caractérisé en ce que** le module externe (12) comporte une unique antenne externe (20), et **en ce que** le module interne (14) comporte une unique antenne interne (24).

4. Dispositif (10) selon l'une quelconque des revendications précédentes, **caractérisé en ce que** chaque antenne (20, 24) est reliée à un circuit d'adaptation d'impédance (22, 32) et que chaque antenne (20, 24) et son circuit d'adaptation d'impédance associé (22, 32) résonnent à une fréquence comprise entre 12 et 14 MHz, plus précisément 13,56MHz.

5. Dispositif (10) selon l'une quelconque des revendications précédentes, **caractérisé en ce que** le module externe (12) et le module interne (14) sont destinés à transférer de l'énergie et des données du module externe (12) vers le module interne (14) de manière simultanée.

6. Dispositif (10) selon l'une quelconque des revendications précédentes, **caractérisé en ce que** le module externe (12) et le module interne (14) sont destinés à transférer des données de l'un vers l'autre de manière bidirectionnelle.

7. Dispositif (10) selon l'une quelconque des revendications précédentes, **caractérisé en ce que** le module externe (12) et le module interne (14) sont destinés à transférer de l'énergie et des données du module externe (12) vers le module interne (14) sur une unique bande de fréquences.

8. Procédé de transfert d'énergie et de données, le procédé étant mis en œuvre au moyen d'un dispositif (10) selon l'une quelconque des revendications précédentes, la tension fournie au circuit intégré interne (28) étant comprise entre 100mV et 5V, la tension fournie au montage électronique de récupération d'énergie étant comprises entre 100mV et 50V.

## Patentansprüche

1. Aktive implantierbare medizinische Vorrichtung (10), die teilweise in einen Subjekt implantiert werden kann, wobei die Vorrichtung, nachdem sie in den Subjekt implantiert wurde, ein externes Modul (12) und ein internes Modul (14) am Subjekt umfasst, wobei das externe Modul (12) und das interne Modul (14) dazu bestimmt sind, Daten untereinander und Energie vom externen Modul (12) zum internen Modul (14) zu übertragen,

   - wobei das externe Modul (12) eine externe Antenne (20) aufweist,
   - wobei das interne Modul (14) Folgendes aufweist:

     ▪ eine interne Antenne (24), die zwei Anschlüsse (241, 242) definiert;
     ▪ eine interne integrierte Schaltung (28), die der internen Antenne (24) zugeordnet ist und eine äquivalente Eingangsimpedanz mit kapazitivem Anteil $C_4$ hat, wobei die interne integrierte Schaltung (28) mit dem ersten Anschluss (242) verbunden ist und mit einem Kondensator mit der Kapazität $C_3$ verbunden ist, wobei der Kondensator mit der Kapazität $C_3$ mit dem zweiten Anschluss (241) verbunden ist, wodurch eine Serienschaltung der internen integrierten Schaltung (28) und des Kondensators $C_3$ zwischen den beiden Anschlüssen (241, 242) gebildet wird, wobei das Verhältnis $\dfrac{C3}{C3+C4}$ größer oder gleich 0,1 und kleiner als 0,7 ist, und

- eine elektrische Schaltung zur Rückgewinnung elektrischer Energie, die mit den Anschlüssen (241, 242) verbunden ist, wobei die elektronische Rückgewinnungsschaltung extern zur internen integrierten Schaltung (28) liegt.

2. Vorrichtung (10) nach dem vorhergehenden Anspruch, **dadurch gekennzeichnet, dass** das Verhältnis $\dfrac{C3}{C3+C4}$ größer oder gleich 0,25 ist und vorzugsweise zwischen 0,3 und 0,6 liegt.

3. Vorrichtung (10) nach einem der vorhergehenden Ansprüche, **dadurch gekennzeichnet, dass** das externe Modul (12) eine einzige externe Antenne (20) aufweist und dass das interne Modul (14) eine einzige interne Antenne (24) aufweist.

4. Vorrichtung (10) nach einem der vorhergehenden Ansprüche, **dadurch gekennzeichnet, dass** jede Antenne (20, 24) mit einem Impedanzanpassungsschaltung (22, 32) verbunden ist und dass jede Antenne (20, 24) und ihre zugehörige Impedanzanpassungsschaltung (22, 32) bei einer Frequenz von 12 bis 14 MHz, genauer gesagt 13,56 MHz, resonieren.

5. Vorrichtung (10) nach einem der vorhergehenden Ansprüche, **dadurch gekennzeichnet, dass** das externe Modul (12) und das interne Modul (14) dazu bestimmt sind, gleichzeitig Energie und Daten vom externen Modul (12) zum internen Modul (14) zu übertragen.

6. Vorrichtung (10) nach einem der vorhergehenden Ansprüche, **dadurch gekennzeichnet, dass** das externe Modul (12) und das interne Modul (14) dazu bestimmt sind, Daten bidirektional untereinander zu übertragen.

7. Vorrichtung (10) nach einem der vorhergehenden Ansprüche, **dadurch gekennzeichnet, dass** das externe Modul (12) und das interne Modul (14) dazu bestimmt sind, Energie und Daten vom externen Modul (12) zum internen Modul (14) über ein einziges Frequenzband zu übertragen.

8. Verfahren zur Energie- und Datenübertragung, wobei das Verfahren mittels einer Vorrichtung (10) nach einem der vorhergehenden Ansprüche umgesetzt wird, wobei die der internen integrierten Schaltung (28) bereitgestellte Spannung zwischen 100 mV und 5 V liegt, wobei die der elektronischen Schaltung zur Rückgewinnung von Energie bereitgestellte Spannung zwischen 100 mV und 50 V liegt.

**Claims**

1. An active implantable medical device (10) able to be partially implanted in a subject, the device comprising, once implanted, an external module (12) to the subject and an internal module (14) to the subject, the external module (12) and the internal module (14) being intended to transfer data from one to the other and energy from the external module (12) to the internal module (14),

   - the external module (12) comprising an external antenna (20),
   - the internal module (14) comprising:

      - an internal antenna (24) defining two terminals (241, 242);
      - an internal integrated circuit (28) associated with the internal antenna (24) having an equivalent input impedance with a capacitive part $C_4$, said internal integrated circuit (28) being connected to the first terminal (242) and being connected to a capacitor with a capacitance $C_3$, said capacitor with a capacitance $C_3$ being connected to the second terminal (241) thereby forming an association in series of the internal integrated circuit (28) and of the capacitor $C_3$ between said terminals (241, 242), with the ratio $\dfrac{C_3}{C_3+C_4}$ greater than or equal to 0.1 and lower than 0.7, and
      - an electrical energy recovery electronic assembly connected to said terminals (241, 242), the recovery electronic assembly being external to the internal integrated circuit (28).

2. The device (10) according to the preceding claim, **characterised in that** the ratio $\dfrac{C_3}{C_3+C_4}$ is greater than or equal to 0.25, preferably comprised between 0.3 and 0.6.

3. The device (10) according to any one of the preceding claims, **characterised in that** the external module (12) includes a unique external antenna (20), and **in that** the internal module (14) includes a unique internal antenna (24).

4. The device (10) according to any one of the preceding claims, **characterised in that** each antenna (20, 24) is connected to an impedance matching circuit (22, 32) and that each antenna (20, 24) and its associated impedance matching circuit (22, 32) resonate at a frequency comprised between 12 and 14 MHz, more specifically 13.56 MHz.

5. The device (10) according to any one of the preceding claims, **characterised in that** the external module (12) and the internal module (14) are intended to transfer energy and data from one to the other simultaneously.

6. The device (10) according to any one of the preceding claims, **characterised in that** the external module (12) and the internal module (14) are intended to transfer data from one to the other in a bidirectional manner.

7. The device (10) according to any one of the preceding claims, **characterised in that** the external module (12) and the internal module (14) are intended to transfer energy and data from one to the other over a unique frequency band.

8. A method for transferring energy and data, the method being implemented by means of a device (10) according to any one of the preceding claims, the voltage supplied to the internal integrated circuit (28) being comprised between 100 mV and 5 V, the voltage supplied to the energy recovery electronic assembly being comprised between 100 mV and 50 V.

[Fig. 1]

[Fig. 2]

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 2018034319 A
- FR 3085551